# EUROPÄISCHE PATENTSCHRIFT

(11) **EP 0 730 471 B1**
(45) Veröffentlichungstag und Bekanntmachung des Hinweises auf die Patenterteilung: **09.05.2001**
(21) Anmeldenummer: 95901441.6
(22) Anmeldetag: 26.11.1994
(51) Int. Cl.: A61K 38/55, A61K 33/14, A61K 33/00

(54) **VERWENDUNG VON LITHIUMVERBINDUNGEN ZUR BEHANDLUNG VON HERZINSUFFIZIENZ**
USE OF LITHIUM COMPOUNDS IN THE TREATMENT OF CARDIAC INSUFFICIENCY
UTILISATION DE COMPOSES DE LITHIUM DANS LE TRAITEMENT DE L'INSUFFISANCE CARDIAQUE

(30) Priorität: 27.11.1993 DE 4340437
(43) Veröffentlichungstag der Anmeldung: 11.09.1996
(73) Patentinhaber: Lehmann, Karla, 70184 Stuttgart (DE)
(72) Erfinder: Lehmann, Karla, 70184 Stuttgart (DE)
(86) Internationale Anmeldenummer: EP9403920
(87) Internationale Veröffentlichungsnummer: WO9514490

(56) Entgegenhaltungen:
- EP-A- 0 068 854
- WO-A-91/17771
- DE-A- 2 410 181
- ORV HETIL (HUNGARY), JAN 16 1983, VOL. 124, NO. 3, PAGE(S) 139-44, Worum I et al 'Litium-karbonat kezelessel szerzett tapasztalataink sulyos, terapia rezisztens cardialis decompensatioban.'
- ACTA MED HUNG (HUNGARY), 1983, VOL. 40, NO. 2-3, PAGE(S) 165-74, Worum I et al 'failure.'
- DATABASE WPI Week 9422, Derwent Publications Ltd., London, GB; AN 94-181775 & SU,A,1 804 842 (AS ARMN CARDIOLOGY RES INST) 30. März 1903
- R. BERKOW ET AL. 'THE MERCK MANUAL' 1987 , MERCK SHARP & DOHME RESEARCH LABORATORIES , RAHWAY, N.J. siehe Seite 415 - Seite 429 siehe besonders Seite 428 - Seite 429

## Beschreibung

Die Erfindung betrifft die Verwendung von Lithiumverbindungen zur Behandlung von Herzinsuffizienz einschließlich ihrer Folgeerkrankungen, insbesondere zur Senkung der dadurch verursachten Herz-Kreislauf-Mortalität.

Herz-Kreislauf-Erkrankungen stehen in Deutschland an der Spitze aller Todesursachen (Bergmann et al., "Daten zur Entwicklung der Mortalität in Deutschland von 1955 bis 1989", Bundesgesundheitsblatt 1: 29-43, 1992). Hauptverursacher für die hohe kardiovaskuläre Mortalität sind chronischer Bluthochdruck und schwere Herzinsuffizienz einschließlich ihrer wechselseitigen Abhängigkeiten und einschließlich ihrer Folgeerkrankungen. Sowohl Bluthochdruck wie auch Herzinsuffizienz werden von einer erhöhten sympathischen (adrenergen) Aktivität und einer erhöhten Aktivität des Renin-Angiotensin-Aldosteron-Systems (RAAS) sowie von Störungen im Elektrolythaushalt des Organismus begleitet. Natriumausschwemmende, antiadrenerge Wirkungen und/oder eine Hemmung des RAAS bzw. eine Reduktion von Angiotensinwirkungen fanden jeweils in Form spezifischer Arzneimittel bereits Eingang in die Behandlung von Bluthochdruck und/oder der Herzinsuffizienz.

Für die schwere finale Herzinsuffizienz ist bekannt, daß es neben der Downregulation betaadrenerger Rezeptoren zu einer Zunahme myokardialer Gi-Proteine kommt, die funktionelle Relevanz besitzt (Neumann et al., "Increase in myocardial Gi-Proteins in heart failure", Lancet 2(8617): 936-937, 1988; Feldman et al., "Altered expression of alpha-subunits of G-Proteins in failing human hearts", J. mol. cell. cardiol. 21(4): 359-365, 1989; Boehm et al., "Beta-Adrenozeptoren und Guanin-Nukleotidbindende Proteine (G-Proteine) am insuffizienten menschlichen Herzen", Z. Kardiol. 81 Suppl. 4: 23-31, 1992; Stiles, G.L., "Adrenergic receptor responsiveness and congestive heart failure", Amer. J. Cardiol. 67(12): 13C-17C, 1991). Da Gi-Proteine die Aktivität der nachgeschalteten Adenylatzyklase im Myokard regulieren, verursacht ihre Zunahme oder eine Verstärkung ihrer funktionellen Aktivität auch eine zunehmende Hemmung der Adenylatzyklase, die sich in negativ inotropen Effekten äußert und bei Aufrechterhaltung zur Progression der Herzinsuffizienz beiträgt. Das Ansprechen auf cAMP-abhängige positiv inotrope Substanzen wird dadurch verringert (Boehm a.a.O.). Eine Reduktion von Gi-Proteinen oder eine Senkung ihrer funktionellen Aktivität verspricht therapeutische Erfolge in der Behandlung von Herzinsuffizienz.

Aus dem Dokument ORV HEITL, 1983, Vol. 124, Nr. 3, Seiten 139 bis 144, und aus dem Dokument Acta Med. Hung., Vol. 40, Nr. 2 bis 3, Seiten 165 bis 174, ist der Einsatz von Lithiumcarbonat zur Besserung von schweren Herzinsuffizienzen beschrieben.

Die Dokumente Database WPI, Week 9422, Derwent Publication Ltd., London, AN 94-181775, und SU,A,1 804,842, AS ARMN CARDIOLOGY RESEARCH, 1993, beschreiben die kardiotonischen Eigenschaften von Lithiumverbindungen, nämlich das Lithimsalz der γ-Aminobuttersäure.

Das Dokument DE,A,24 10 181 betrifft die Verwendung von Lithiumorotat zur Behandlung von Gefäß- und Gewebserkrankungen, insbesondere im cerebralen Bereich, sowie Hypertonie, Arteriosklerose, Alterung und Gewebsverschleiß sowie Migräne.

Das Dokument EP,A,0 688,854 beschreibt die Herstellung eines Arzneimittels bestehend aus Linolensäure und einem Lithiumsalz für die Behandlung von ischämischen Herzkrankheiten.

Aus den Dokumenten Merck Manual, 15th Edition, Berghoff et al., 1987, und aus dem Dokument WO,A,91 17771 ist es bekannt, Herzinsuffizienz mit einem ACE-Hemmer und/oder einem Angiotensin II-Rezeptor-Antagonisten zu behandeln.

Aufgabe der vorliegenden Erfindung ist es, neue Möglichkeiten zu schaffen, um die Herzinsuffizienz einschließlich ihrer Folgeerkrankungen, insbesondere zur Senkung der dadurch verursachten Herz-Kreislauf-Mortalität zu finden.

Erfindungsgemäß wird die Aufgabe durch die Verwendung von Lithiumverbindungen in fixer Kombination mit zumindest einem ACE-Hemmer und/oder in fixer Kombination mit zumindest einem Angiotensin II-Rezeptor-Antagonisten zur Herstellung eines Arzneimittels zur Behandlung von Herzinsuffizienz einschließlich ihrer Folgeerkrankungen, insbesondere zur Senkung der dadurch verursachten Herz-Kreislauf-Mortalität gelöst.

Durch das umfassende Wirkungsspektrum von Lithium, insbesondere durch die Senkung adrenerger (sympathischer) Funktionen, die Hemmwirkung auf das RAAS, durch Natriurese und durch eine Stabilisierung von Gi-Funktionen, werden die zuvor aufgeführten Ursachen der Herzinsuffizienz bekämpft. Lithium führt in therapeuthisch wirksamen Dosen (20-50 mmol/Tag) zur Abnahme der pressorischen Ansprechbarkeit auf Angiotensin beim Menschen. Lithium erhöht in therapeutisch wirksamen Dosen (beispielsweise 32 mmol) die zu einer Blutdrucksteigerung notwendigen Noradrenalin-Dosen beim Menschen. Lithium hemmt im Gegensatz zu zweiwertigen Ionen, wie Mangan, Magnesium oder Kalzium, die hochaffine Bindung von Angiotensin II an peripheren Gefäßrezeptoren. Intrazellulär hemmt Lithium einerseits die für die Vermittlung adrenerger sympathischer Effekte wichtige Adenylatzyklase. Andererseits senkt Lithium über eine nichtkompetitive Hemmung der Monophosphatase den Abbau von Inositphosphaten zu Inosit. Längerfristig resultiert aus dieser Lithiumwirkung eine Depletion von IP₃, dem wichtigsten Vermittler der intrazellulären Angiotensin II-Wirkungen, wodurch weitere angiotensinerge Effekte reduziert werden. Lithium löst außerdem eine nachweisbare Natriurese aus und kann Natrium, das blutdrucksteigernd und herzbelastend ist, im Organismus ersetzen. Unterstützt werden diese Effekte durch eine lithiumbedingte Hemmung der vasopressinabhängigen Wasserreabsorption im distalen Tubulus der Niere, woraus eine zusätzliche Kreislaufentlastung resultiert. Es konnte nach subakuter Applikation von Lithium eine Abschwächung von Gi-Funktionen in Blutplättchen von Probanden und im Rattenkortex nachgewiesen werden. Vermutet wird eine Stabilisierung von Gi-Proteinen in undissoziierter inaktiver Form.

Erste Untersuchungen an Patienten, denen Lithiumverbindungen in therapeutischen Dosen verabreicht wurden, haben ergeben, daß dadurch Ursachen der Herzinsuffizienz wirksam bekämpft werden können.

In Dosen im Bereich derer, die bei anderen Indikationen Verwendung finden, sind unerwünschte Reaktionen von Lithiumverbindungen selten und begrenzbar. Die umfangreichen Erfahrungen, die im Umgang mit Lithiumverbindungen bei anderen Indikationen über Jahrzehnte hinweg gewonnen wurden, sind für die sichere Langzeitanwendung bei Patienten mit Herzinsuffizienz und dadurch verursachten Herz-Kreislauf-Erkrankungen und/oder bei Risikopatienten nutzbar. Insgesamt erweist sich demzufolge Lithium als ideales und physiologisches Arzneimittel zur Senkung der Herz-Kreislauf-Mortalität bei gleichzeitiger Bekämpfung ihrer Ursachen.

Als Lithiumverbindungen können besonders vorteilhaft solche eingesetzt werden, die ausgewählt sind aus der Gruppe bestehend aus Lithiumazetat, -adipinat, -aspartat, -hydrogenaspartat, -benzoat, -bromid, -chlorid, -glukonat, -karbonat, -orotat, -salizylat, -sulfat, -zitrat.

Die Addition des zweiten Wirkstoffes gegen die Herzinsuffizienz verbessert den Effekt und erhöht die Ansprechquote der Behandlung. Durch die erfindungsgemäße fixe Kombination der Lithiumverbindung mit einem ACE-Hemmer und/oder einem Angiotensin II-Rezeptor-Antagonisten können alle bekannten Ursachen der Herzinsuffizienz bekämpft werden, bei gleichzeitiger verbesserter und verbreiterter Wirksamkeit der ACE-Hemmer. Bei Kombination beider Wirkstoffe, nämlich Lithiumverbindung und ACE-Hemmer, kann die übliche Dosierung gesenkt werden, wodurch die Verträglichkeit des ACE-Hemmers verbessert wird, die bislang ein großes Problem bei der ACE-Hemmer-Therapie dargestellt hat.

Dabei hat sich als besonders vorteilhaft die Verwendung von Alacepril, Benazepril, Captopril, Cilazapril, Delapril, Enalapril, Fentiapril, Fosinopril, Lisinopril, Moveltripril, Pentopril, Perindopril, Pivalopril, Ramipril, Quinapril, Spirapril, Zofenopril als ACE-Hemmer herausgestellt.

Auch in der fixen Kombination von Lithiumverbindungen mit Angiotensin II-Rezeptor-Antagonisten konnte eine verbesserte und verbreiterte Wirksamkeit festgestellt werden.

Es sind auch Anwendungen von Lithiumverbindungen sowohl mit ACE-Hemmern als auch mit Angiotensin II-Rezeptor-Antagonisten möglich.

Das molare Verhältnis der Lithiumverbindung zu den ACE-Hemmern und/oder Angiotensin II-Rezeptor-Antagonisten kann zwischen 100 bis 0,01 betragen, wobei das Verhältnis so abzustimmen ist, daß daraus eine Erhöhung der Behandlung der Herzinsuffizienz gegenüber der Wirksamkeit der Einzelkomponenten resultiert. Dabei wird auf den molaren Lithiumgehalt des entsprechenden Salzes bezogen.

In zweckmäßiger Weise übersteigt in den erfindungsgemäßen Arzneimitteln die Dosierung der Lithiumverbindungen anderweitig übliche Tagesdosen nicht. Die Dosierung wird auf den molaren Lithium-Gehalt bezogen und ist der entsprechenden Zielgröße anzupassen.

Die erfindungsgemäßen Arzneimittel können in Form systemisch verabreichbarer, insbesondere als oral und/oder intravenös verabreichbare Formulierungen konfektioniert sein.

Lithiumverbindungen können in üblichen pharmazeutischen Trägern, Verdünnungsmitteln und/oder Hilfsstoffen verwendet werden.

## Patentansprüche

1. Verwendung von Lithiumverbindungen in fixer Kombination mit zumindest eine Angiotensin-Converting-Enzym (ACE)-Hemmer und/oder in fixer Kombination mit zumindest einem Angiotensin II-Rezeptor-Antagonisten zur Herstellung eines Arzneimittels zur Behandlung von Herzinsuffizienz einschließlich ihrer Folgeerkrankungen, insbesondere zur Senkung der dadurch verursachten Herz-Kreislauf-Mortalität.

2. Verwendung nach Anspruch 1, dadurch gekennzeichnet, daß der zumindest eine ACE-Hemmer ausgewählt ist aus der Gruppe bestehend aus Alacepril, Benazepril, Captopril, Cilazapril, Delapril, Enalapril, Fentiapril, Fosinopril, Lisinopril, Moveltipril, Pentopril, Perindopril, Pivalopril, Ramipril, Quinapril, Spirapril, Zofenopril.

3. Verwendung nach Anspruch 1 oder 2, dadurch gekennzeichnet, daß als Angiotensin II-Rezeptor-Antagonist Losartan oder wirksame Metaboliten der Angiotensin II-Rezeptor-Antagonisten verwendet werden.

4. Verwendung nach einem der Ansprüche 1 bis 3, dadurch gekennzeichnet, daß die Wirkstoffe in Form systemisch verabreichbarer, insbesondere als oral und/oder intravenös verabreichbare Formulierungen konfektioniert sind.

5. Verwendung nach einem der Ansprüche 1 bis 4, dadurch gekennzeichnet, daß die Wirkstoffe in üblichen pharmazeutischen Trägern, Verdünnungsmitteln und/oder Hilfsstoffen verwendet werden.

6. Verwendung nach einem der Ansprüche 1 bis 5, dadurch gekennzeichnet, daß das molare Verhältnis zwischen Lithiumverbindung und ACE-Hemmer bzw. Angiotensin II-Rezeptor-Antagonist zwischen 100 und 0,01 liegt.

## Claims

1. Use of lithium compounds in a fixed combination with at least one angiotensin converting enzyme (ACE) inhibitor and/or in a fixed combination with at least one angiotensin II receptor antagonist for the preparation of a drug for treating cardiac insufficiency, including its secondary diseases, and especially for reducing the cardiovascular mortality caused thereby.

2. Use according to Claim 1, characterized in that the ACE inhibitor(s) is (are) selected from the group consisting of alacepril, benazepril, captopril, cilazapril, delapril, enalapril, fentiapril, fosinopril, lisinopril, moveltipril, pentopril, perindopril, pivalopril, ramipril, quinapril, spirapril and zofenopril.

3. Use according to Claim 1 or 2, characterized in that the angiotensin II receptor antagonist used is losartan or an active metabolite of an angiotensin II receptor antagonist.

4. Use according to one of Claims 1 to 3, characterized in that the active substances are formulated for systemic administration, especially oral and/or intravenous administration.

5. Use according to one of Claims 1 to 4, characterized in that the active substances are used in conventional pharmaceutical excipients, diluents and/or auxiliary substances.

6. Use according to one of Claims 1 to 5, characterized in that the molar ratio of lithium compound to ACE inhibitor or angiotensin II receptor antagonist is between 100 and 0.01.

## Revendications

1. Utilisation de composés de lithium en combinaison fixe avec au moins un inhibiteur de l'ECA (enzyme de conversion de l'angiotensine) et/ou en combinaison fixe avec au moins un antagoniste du récepteur de l'angiotensine II dans la préparation d'un médicament pour le traitement de l'insuffisance cardiaque, y compris ses séquelles, en particulier pour abaisser la mortalité cardio-vasculaire qui en résulte.

2. Utilisation selon la revendication 1, caractérisée en ce que l'inhibiteur de l'enzyme de conversion de l'angiotensine est choisi dans le groupe comprenant l'alacépril, le benazépril, le captopril, le cilazapril, le délapril, l'énalapril, le fentiapril, le fosinopril, le lisinopril, le moveltipril, le pentopril, le perindopril, le pivalopril, le ramipril, le quinapril, le spirapril, le zofénopril.

3. Utilisation selon la revendication 1 ou 2, caractérisée en ce que l'on utilise comme antagoniste du récepteur de l'angiotensine II le losartan ou des métabolites actifs des antagonistes du récepteur de l'angiotensine II.

4. Utilisation selon l'une quelconque des revendications 1 à 3, caractérisée en ce que les principes actifs sont formulés en compositions pouvant être administrées par voie systémique, notamment administrées par voie orale et/ou intraveineuse.

5. Utilisation selon l'une quelconque des revendications 1 à 4, caractérisée en ce que les principes actifs sont utilisés dans des véhicules, des diluants et/ou des adjuvants pharmaceutiques classiques.

6. Utilisation selon l'une quelconque des revendications 1 à 5, caractérisée en ce que le rapport molaire du composé de lithium à l'inhibiteur de l'enzyme de conversion de l'angiotensine ou de l'antagoniste du récepteur de l'angiotensine II varie de 100 à 0,01.
